(19) Europäisches Patentamt
European Patent Office
Office européen des brevets

(11) EP 3 423 617 B1

(12) # EUROPEAN PATENT SPECIFICATION

(45) Date of publication and mention of the grant of the patent:
**10.04.2019 Bulletin 2019/15**

(21) Application number: **16809550.3**

(22) Date of filing: **28.09.2016**

(51) Int Cl.:
***D01F 2/00*** *(2006.01)*

(86) International application number:
**PCT/TR2016/050358**

(87) International publication number:
**WO 2017/155487 (14.09.2017 Gazette 2017/37)**

(54) **PRODUCTION OF WATER-SOLUBLE REGENERATED FIBER FROM CALLUNA VULGARIS PLANT SPECIES**

HERSTELLUNG VON WASSERLÖSLICHER REGENERIERTER FASER AUS PFLANZEN DER GATTUNG CALLUNA VULGARIS

PRODUCTION DE FIBRE RÉGÉNÉRÉE SOLUBLE DANS L’EAU À PARTIR DE L’ESPÈCE VÉGÉTALE CALLUNA VULGARIS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.03.2016 TR 201602906**

(43) Date of publication of application:
**09.01.2019 Bulletin 2019/02**

(73) Proprietor: **Veritas Tekstil Konfeksiyon Pazarlama Sanayi ve Ticaret Anonim Sirketi**
**Honaz/Denizli (TR)**

(72) Inventor: **SOYLU, Mustafa**
**Denizli (TR)**

(74) Representative: **Yamankaradeniz, Kemal**
**Destek Patent, Inc.**
**Eclipse Business D**
**Blok No. 5 Maslak**
**34398 Istanbul (TR)**

(56) References cited:
**EP-A1- 1 354 914**
**WO-A1-2014/041251**
**US-A1- 2002 155 292**
**US-A1- 2009 165 969**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 423 617 B1

## Description

### The Related Art

The invention relates to production of regenerated fiber from cellulose-containing plant species, in textile sector. The invention particularly relates to obtaining water-soluble regenerated cellulose fiber using high or low purity cellulose raw material isolated from the wild plant species of Calluna Vulgaris.

### The Prior Art

Throughout history, people have committed themselves to do studies that facilitate their lives more and bring innovations all the time. Petrochemistry products that are found almost every part of human life since industrial revolution until today compelled people to look for alternative sources due to environmental problems they cause due to remaining in nature for many years without decomposition as well as a number of health problems. Today, it has become necessary to increase raw material resources of regenerated fibers that are derived from cellulose that is the most convenient material for use in nature and to offer diversity in man-made fiber production. Regenerated fibers that are a type of chemical fibers are derived chemically from natural raw materials. Natural raw materials can be used in textiles by reshaping through chemical processes or turned into filament or staple fiber.

If cellulose is used as natural polymer that constitutes regenerated fiber, it is called regenerated cellulosic fiber. Rayon and floss are the names attributed to regenerated cellulosic fiber in filament form. They are the most important representatives of natural origin man-made fiber production.

While production of (dissoluble) chemical pulp that is rich in alpha-cellulose varied between 4,6 and 5,0 million tons until 1980’s, around 1,4 million tons were produced in North America. Since then, production amount decreased to 2,7 million tons due to either production of alternative products at lower cost for the present or outdated technology of current established systems.

In the prior art, water-insoluble yarn is derived through conventional regenerated cellulose fiber production methods. Hence, there is a need for obtaining water-soluble yarn by using natural raw material source and renewable cellulose source.

Nowadays, water-soluble filament yarns are derived from polyvinyl alcohol polymers. Polyvinylacetate that is derived by means of polymerization reaction of vinylacetate is processed with caustic and composes polyvinylalcohol polymer. Filament yarn is derived from polyvinylalcohol (PVA) polymers that are obtained in this way by means of wet or dry fiber spinning methods.

Recyclability is a required specification for products today. Water-soluble Polyvinylalcohol (PVA) yarn is removed by treating it with formic acid, acetic acid, or citric acid at 100 degrees in dyeing plant. Dissolved PVA polymer cannot be recycled as it is split into formaldehyde and acetaldehyde groups.

Bath temperature needs to be raised to 100°C in order to dissolve PVA during dyeing-finishing processes of raw fabric that is derived from PVA yarns and pliable cotton yarns. In the meantime, PVA polymer forms sediments by sticking to the product at rapidly increasing bath temperatures and direct steam contacts. On the other hand, vinyl acetate that is raw material of water-soluble conventional PVA yarn is one of the xenobiotic organic compounds that are widely used in chemical industries. Therefore, this compound is required to be removed efficiently and economically from waste water and waste gases in chemical industries.

Xenobiotic compounds are industrial synthetic compounds released to environment at high concentrations. The term "xenobiotic" is described as substrate or biochemical that is foreign to biologic system or organism. Lexical meaning of xeno is foreign. Xenobiotic chemicals are produced in a large number of chemical industries. In recent years, substantial amount of such chemicals are released to environment freely. Benzene, toluene, styrene, xylene, and ethyl benzene are among the most common industrial aromatic products millions of tons of which are produced a year. Xenobiotics are gradually increasing in urban water supply systems due to increase in chemical pollution, pesticide applications, industrial production, use of domestic chemicals, traffic emulsions, and pharmaceutical applications. Xenobiotics also comprise inorganic elements such as heavy metals, metalloids, surfactants, and preservative substances. While modern chemical industry provides society with significant benefits, it also causes negative impacts due to release of xenobiotics to environment. Vinyl acetate falls into the group of volatile organic compounds and is included in the list of organic hazardous air pollutants.

The first patent related to polyvinylalcohol production was taken out by W.O. Herman and W. Haehnel in 1931. However, polyvinylalcohol fibers came to draw interest in Japan and currently it is mostly produced in Japan. Below is a reaction related to obtaining polyvinylalcohol.

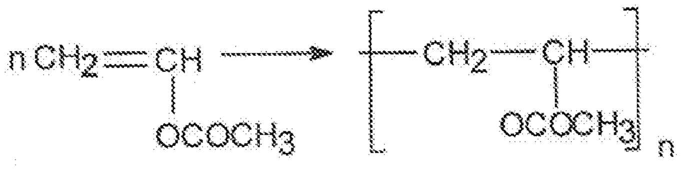

vinylacetate polyvinylacetate

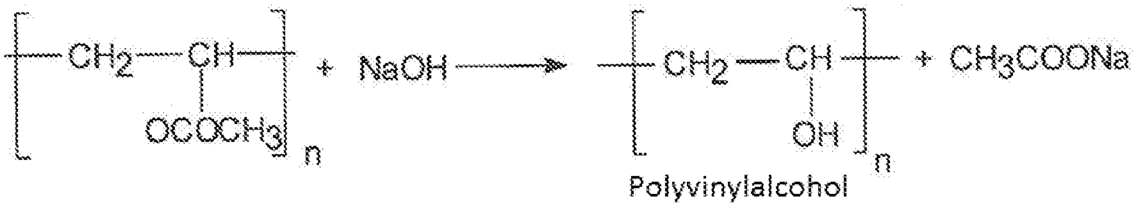

Polyvinylalcohol

There are two ways of fiber spinning through polyvinylalcohol (PVA) polymers obtained by the abovementioned reaction.

***Wet fiber spinning method:*** 14-16 % hot aqueous solution of polyvinylalcohol polymers is prepared. This solution is enabled to solidify by being sprayed from a nozzle and passing through the precipitation bath comprising $Na_2SO_4$ and $(NH_4)_2\,SO_4$.

***Dry fiber spinning method:*** Concentrated aqueous solution of polyvinylalcohol polymers is prepared. The water in the solution that is sprayed from the nozzles is enabled to evaporate (130°C) and the fibers are solidified. Hot dry air is utilized to vaporize water.

Polyvinylalcohol fibers that are obtained according to both methods are easily water-soluble. Although this property enables the fibers to be used as catgut in medicine, they must be made water-insoluble for use in textile. For this, the fiber is first subjected to hot processing at 210-230°C for about 20 seconds and bridge connections are established between macromolecules. Then it is passed through hardening baths comprising formaldehyde and enables 40 % of hydroxyl groups to get into acetate or ketolisation reactions. Thus, while hydroxyl groups that enable dissolving in water are eliminated, bridge connections are also established.

The reactions that occur during hot processing and hardening are as follows.

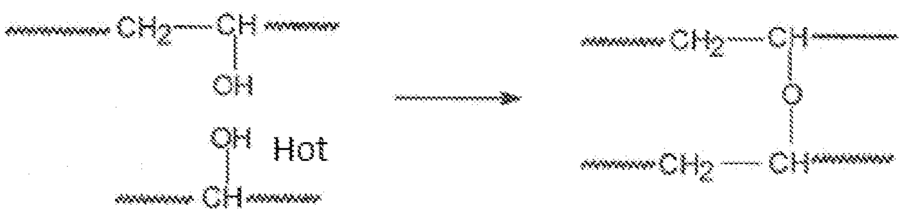


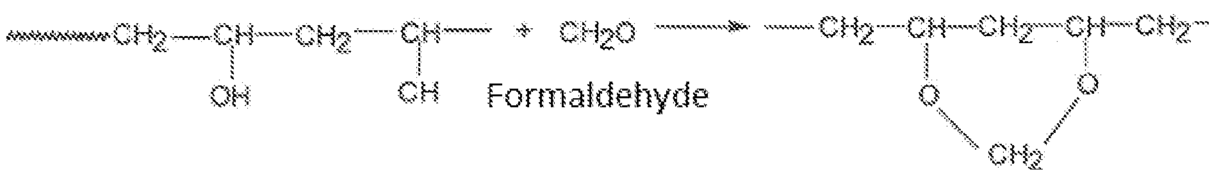


or

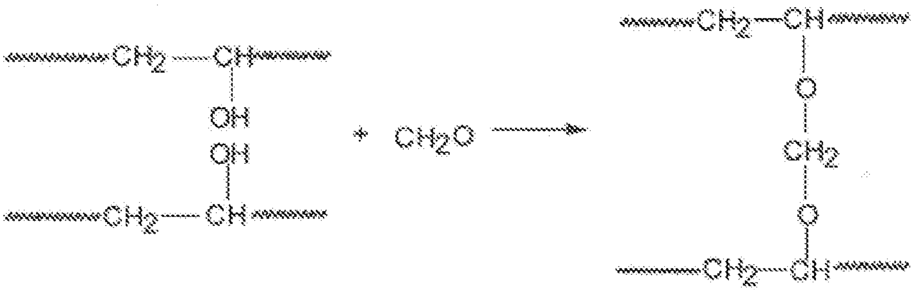

***Physical properties:*** They are white and shiny. Wet breaking strength of high breaking strength filamets are 5-7,6 g/denier, and their dry breaking strength are 6-8,5 g/denier, and their elongation rate is between 10-26 % when wet, and 9-22 % when dry.
Wet breaking strengths of staple fibers are 3-2.5 g/denier, and 3,8-6,2 g/denier when dry, and their elongation rate is 16-27 % when wet, and 13-26 % when dry. Specific weigth of polyvinylalcohol fibers is around 1,26-1,30 g/cm$^3$.
In the prior art, there are three types of chemical fiber spinning methods as

- Wet spinning
- Dry spinning
- Soft spinning.

In dry spinning method, the solvents to be used to prepare polymer solution must be volatile, in other words, they must be a substance that have low boiling point. When such a solution is sprayed from nozzles under constant pressure to the chambers through which warm air current passes, the solvent is easily evaporated and the polymer substance that is filament-formed remains. Acetate, triacetate and acrylic fibers are obtained by dry spinning method.
In soft spinning method, the polymers that are not dissolved in any solvent and have thermoplastic (thermoform) property are turned into filaments. In this method, the polymer substances in chips form are liquefied (molten) at a temperature above melting point. Molten polymer is sprayed by means of a pump from nozzle heads to the chambers through which cold air current passes under constant pressure. Molten polymer is solidified in filament form in cold chambers. Polyamide, polyester, and polyurethane fibers are obtained by soft spinning method.
Viscose fiber is typically obtained in two ways.

- Viscose rayon (in filament form)
- Viscose (in staple form)

To produce viscose, the cellulose raw material obtained from wood and linter is cleared of foreign substances by processing it with caustic soda and sodium bisulphite. Cellulose pulp is turned into alkali cellulose by processing it with caustic soda solution (NaOH). Cellulose xanthate is obtained by adding carbon sulphur ($CS_2$) to alkali cellulose following pre-ripening process. It is turned into raw viscose solution with the addition of diluted sodium hydroxide. Viscose solution is sprayed from the nozzles to an acid bath following filtering and post-ripening process, and then solidified viscose filaments are obtained by wet spinning method. The filament obtained from single-hole nozzle head is called "monofilament", the one obtained from multi-hole nozzle head is called "multifilament". Viscose rayon yarn is obtained by wrapping solidified filaments on the coil following stretching, washing, and drying processes.
Another method is Lyocell method. In Lyocell method, hot solvent dissolves lignin under simultaneous water vapour and intense cutting. This type of production is highly clean and viscose solution can be extruded in diluted solution. This solvent is recovered by dissolving without spinning and washing. Effective solvent recovery is a key criteria to reduce the cost and for a successful process, and fibers can be produced in filament and staple form that has circular section. Companies such as Lenzing, Couraulds use NMMO as solvent.
Three possible forms of N-methyl morpholine-N-oxide (NMMO) are as follows.

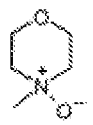

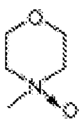

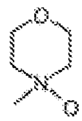

It is followed a shorter route compared to conventional viscose rayon system from the extract to the fiber, and the main difference therein is observed in regeneration. Below is the reaction of hydrogen bonds between macromolecules of cellulose with NMMO organic solvent.

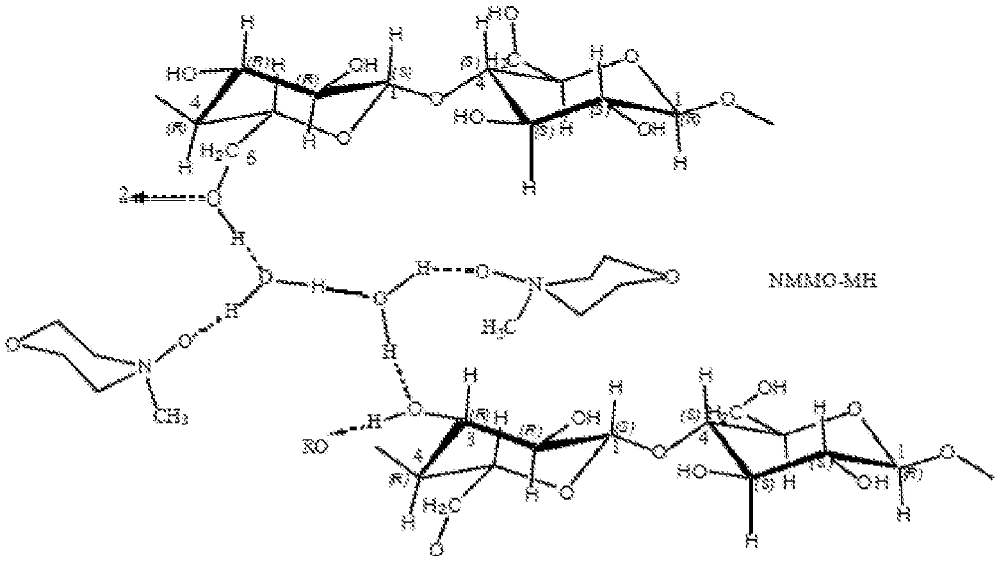

Plant cell and wall is composed of three main components. They are lignin, cellulose, and hemicelluloses. The structure that forms plant stem is the structure sequenced in matrix form. Tree trunks are composed of lignin, cellulose, and hemicelluloses. Cellulose is composed of glucose units sequenced in chain form. They are first sequenced side by side in chain bundles. Then, they compose cell layers. Hemicelluloses, together with cellulose, are sequenced amorphously beside it. While lignin is formless, it covers both celluloses and hemicelluloses.

Wood cells are interconnected with lignin at the middle layer area that is rich in lignin. The amount of lignin therein is around 25-30 % of total lignin. Primary cell wall is composed of randomly sequenced cellulose-based microfilaments. Both lignin and cellulose are together named as middle layer.

Secondary cell wall is composed of 3 layers. The main layer is found between the two thin layers. Their thickness varies by cell type and season. There is one more innermost rough layer in some cells. As the main layer is thick, sequence of its microfilaments is of importance for cellulose. This alignment in cross direction is important in terms of mechanical and physical strength of fiber. This direction in cellulose is called microfilament angle.

While cellulose chain rings compose the chain by side-by-side sequence on secondary section of the cell wall, the chains are also sequenced in layers through side-by-side interconnection. Then, layers are sequenced in overlapping layers. Two different structures are seen in layer sequence due to glucose it comprises. These structures are called alpha cellulose and beta cellulose. While axes of the crystals on the layers of alpha cellulose are sequenced on the same line, they are sequenced in an obfuscatory manner in beta cellulose.

It is possible to see both sequences in cellulose. This is also called sequence of cellulose crystals. While alpha sequence is a form of metastable sequence, beta sequence has more stable structure. All alpha sequences may turn into beta sequence under high pressure and at high temperature, or in acidic or alkali environment.

95 % of the lignin is removed from the wood in which found lignin, cellulose, hemicellulose polymers during extraction processes. Albedo of wood pulp increases depending on removal of other coloured pigments and lignin amount from it while bleaching. Subjecting the pulp to a series of bleaching processes with chemical substances such as chlorine, alkali, hypochlorite, and chlorine dioxide was followed by disintegrating lignin with oxygen and adding it to the process after kiering it with chemical substances and before bleaching in 1970's. In bleaching processes, main objective is to develop processes that reduce bleaching costs and minimize environmental effects.

Major bond structures in lignin are given below.

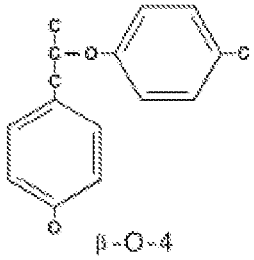
β-O-4

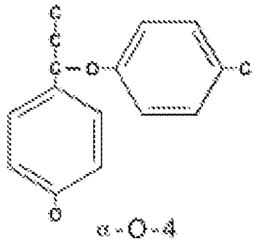
α-O-4

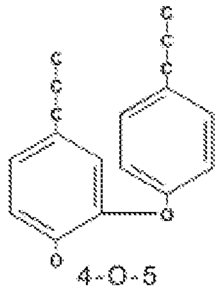
4-O-5

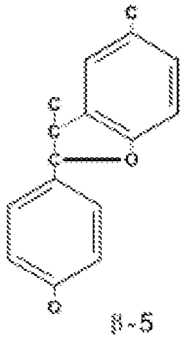
β-5

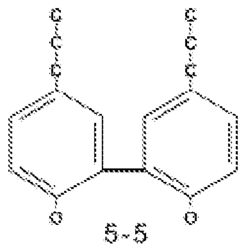
5-5

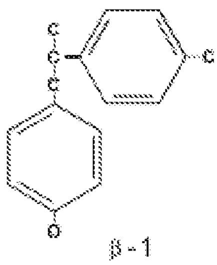
β-1

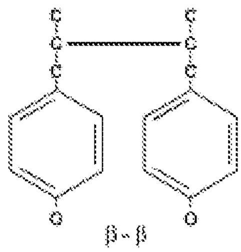
β-β

*Lignin disintegration reactions:*

Chips are kier boiled in 20-30 meter high grinding containers at 170°C for 30 minutes. They are fixed to 140-180°C under 10 atm pressure. Lower temperature of grinding container is fixed to 65°C.

Lignin is disintegrated following kier boiling process. Lignin disintegration reactions are given below. Lignin is also shown as the abbreviation R-R' owing to the fact that there are a wide variety of phenol compounds with large amount of carbon and hydrogen and also phenol and benzene derivatives in its structure.

$$R\text{-}R' + NaOH \rightarrow R''COONa + ROH$$

(Reaction of caustic with lignin compounds) (Lignin : R-R')

$$R\text{-}R' + Na_2S \rightarrow \text{Mercaptans} : H_2S; NaSH, Na_2S$$

(disintegration reaction with lignin and sodium sulphite)

(a) Chemical recovery (Sodium carbonate is derived from the reaction of sodium phenols that are formed as a result of the disintegration with oxygen, and caustic is derived from this compound and used in disintegration of lignin in 1st reaction again.)

$$2NaR + O_2 \rightarrow Na_2CO_3 + CO_2$$

(NaR: Lignin)

(b) Causticization

$Na_2CO_3$ (aq) + $Ca(OH)_2$ (s) → 2NaOH (aq) + $CaCO_3$ (s)

$CaCO_3$→CaO + $CO_2$

CaO + $H_2O$ →$Ca(OH)_2$

*Kappa Number:* It is the rate of lignin that remains in craft cellulose pulp after chemical processes. TAPPI-T236 om-99 method employed to determine kappa number is a method for quantifying the amount of lignin. This method is employed to determine the degree of relative hardness or bleaching or delignification degree of the pulp. Kappa number is calculated by consumption of the solution by volumetric titration method using 0,1 N potassium permanganate solution at moisture-free pulp under the conditions specified in the method. Low kappa number indicates that there is small amount of lignin in the cellulose. Large number indicates that there is large number of lignin.

Kappa number is analyzed upon disintegration of lignin and bleaching process. Kappa number relates to quality. It is important that kappa number is low for suitable viscosity in fiber spinning.

In the second half of 1980's and the first half of 1990's, bleaching process shifted towards application of chlorine gas and hypochlorite. Elemental free chlorine is single-chlorine based and is abbreviated as ECF. In 1990's it was introduced as TCF or Total Free Chlorine. This ordering, hydrogen peroxide, and ozone processes are added to increase effectiveness in bleaching process. Later on, oxygen delignification (lignin degradation) and ozone bleaching processes are added as new processes to minimize the effects of environmental wastes, thus pollution is minimized. Lignin disintegration process using oxygen comprises the use of oxygen and alkali to remove a substantial part of lignin after the kiering process. Low kappa number is reached by using oxygen. Consequently, waste water load of the plant is reduced.

Lignin disintegration process using oxygen occurs at two stages as specified in Table 1 below. Lignin is disintegrated through oxidization with oxidation process following alkali (that is caustic) process.

Table 1: Lignin disintegration process using oxygen

| O2: | Stage 1 | Stage 2 |
|---|---|---|
| Application time | 30 min. | 60 min. |
| Temperature | 80-85 °C | 90-95 °C |
| Density | ≥ 11 % | ≥ 11 % |
| Pressure | 8-10 bar | 3-5 bar |
| Final pH | | 10.5- 11 |

Bleaching processes are covered by TCF and mitigated ECF processes at 80 - 100°C in a pressurized reactor using peroxide. TCF is the abbreviation of the bleaching process applied without using chlorine dioxide ($ClO_2$) compound, ECF (elemental chlorine free) is the abbreviation of the bleaching process applied without using chloride ($Cl_2$) gas. $Cl_2$ (chloride) or $ClO_2$ (chlorine dioxide) compounds are highly reactive chemicals that are used in bleaching processes, and their use causes toxic and contaminant wastes. Chlorine or chlorine dioxide compounds react to lignin, form chlorinated organic compounds and mix in waste water. Use of peroxide, oxygen and ozone chemicals as well as enzymatic reactions gained importance in bleaching process for environmental reasons.

When the reactions that lead to bleaching in peroxide bleaching are analysed, it is found that the ions having the main bleaching effect are $HO_2$ and some $O_{2:}$

$H_2O_2$ + $OH^-$ → $H_2O$ + $HO_2^-$

$H_2O_2$ + $2OH^-$ → $2H_2O$ + $O_2$

A part of hydrogen peroxide is disintegrated itself apart from bleaching reactions. This increases the consumption of hydrogen peroxide. Therefore, such reactions need to be prevented.

Prevention of disintegration depends on;

- pH
- Temperature
- Time and

- Stabilizer effect.

The purpose of using stabilizer is to prevent such undesirable reactions.

Hydrogen peroxide stabilizers are known as Sodiumtripolyphosphate (STPP), Sodiumhexametaphosphate (SHMP), Ethylenediaminetetraaceticacide (EDTA), Sodiumsilicate ($Na_2O.SiO_2$), Magnesiumsilicate ($MgO.SiO_2$). $H_2O_2$ process conditions are given in Table 2 below.

Table 2

| $H_2O_2$ Process | |
|---|---|
| pH value | 9.5- 11 |
| Temperature | 80 -100°C |
| Pulp density | ≥ 11 % |
| Time | 60-120 min. |
| Pressure | 3-5 bar |

In order for the reaction in $H_2O_2$ processes to occur at optimal and efficient level, the reaction environment specified in Table 2 above must be ensured. The pulp solution with concentration above 11 % is treated with EDTA or the abovementioned stabilizers for 60 to 120 minutes. For this reaction environment, pH value must be 9,5 -11, the temperature must be 80-100°C, and the pressure must be 3-5 bar.

*Applying the hemicellulose enzyme xylanase:*

Xylanase enzymes are used in reservoir towers to reduce consumption of bleaching agents. Application areas and working conditions of enzymes depend on certain conditions. For Xylanase enzyme, pH 5-9 and temperature between 60 °C - 90 °C is considered as an application area. Disintegration of hemicellulose is ensured by applying Xylanase enzyme to the pulp at the specified pH and temperature.

Hemicellulose structures are derivatives of heteropolysaccharides comprised of β-(1-4)-D-xylopyranosyl units between lignin and cellulose. Common name of polymer structures such as xylane, glucuronoxylane, arabinoxylane, glucomannan, and xyloglucane is hemicellulose. They form a chain by being bonded with covalent bond at C2-C3 position. Hemicellulose polymers (DP Polymerization degree: 150-250) have highly amorphous and irregular branching; they are more sensitive to reactions compared to cellulose sequenced in straight chains. Xylanhemicellulose, polymer β-(1-4) -D-xylopyranosyl units are given below.

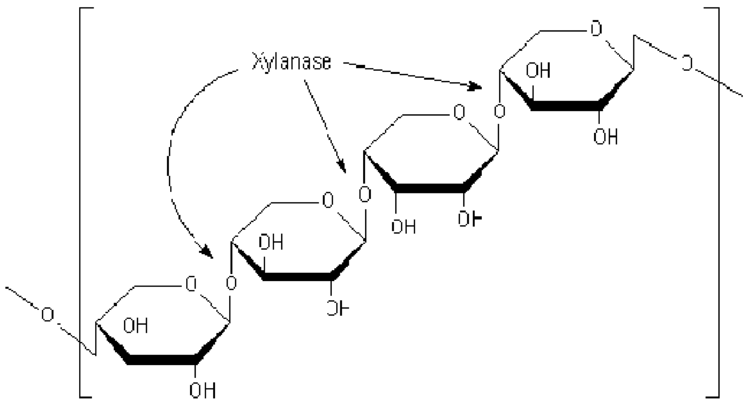


Xylanase enzyme attacks 2nd and 3rd carbons at β-1-4 position in hemicellulose chain and dissolves it into 5-carbon or 6-carbon monosaccharides. 5-carbon carbohydrates arise from hemicellulose Xylanase enzyme reaction as shown below.

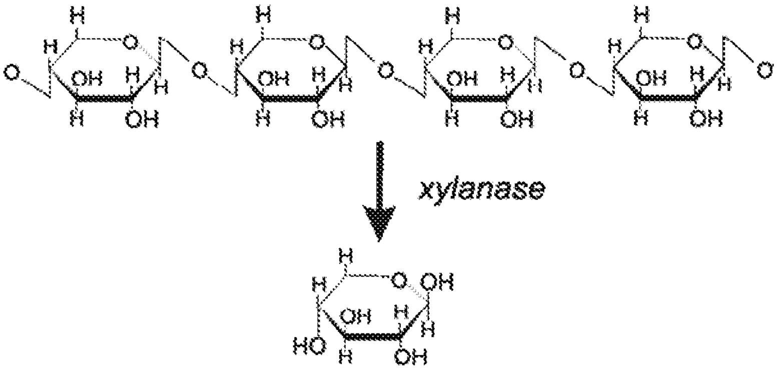

Enzyme activity refers to activity of the enzyme that changes 1$\mu$mol substrate in 1 minute and is considered as a unit.

Another method employed to determine bleaching degree of the pulp that is treated after the reactions is ISO 2470:1999 method. In this method, R rate known as the distribution function measured with reflectometry that is effective at 44nm - 457nm wavelength and has internal reflection indicates the percentage of the reflected radiation. A paper or plate has light absorption (**k**) and distribution (**s**) coefficients. Albedo is a function of **k**/**s**. In bleaching process, k remains constant as **s** decreases and bleaching degree increases.

In the prior art, the first step of regenerated fiber obtaining method comprises removing lignin compounds from the wood structure. In this step, while a series of processes mentioned above can be applied separately to remove lignin compounds, they can also be used in combination to achieve the desired bleaching and reduce environmental waste effects. In wood pulp obtaining processes, Enzymatic (Xylanase) processes are applied in Caustic, ECF (Disintegration of lignin with ozone and oxygen), TCF (Disintegration of lignin with ozone and oxygen), and hemicellulose disintegration processes that do not contain chlorine element or chlorine dioxide compounds mentioned above in order to reduce environmental waste effects, and then bleaching degree is adjusted by means $H_2O_2$ process. In the second step, regenerated fiber is obtained by clearing the wood pulp solution of lignin compounds and treating it with N-methyl morpholine-N-oxide solvent.

## Brief Description of the Invention

The invention relates to a method of obtaining water-soluble regenerated fiber from the plant species of Calluna Vulgaris, which meets the above said requirements, eliminates all of the drawbacks, and brings some additional advantages.

The primary purpose of the invention is to obtain water-soluble polymer fibers with the method of obtaining regenerated cellulose fiber using a renewable cellulose source.

A purpose of the invention is to provide a method to obtain yarns from Calluna Vulgaris (L.) Hull and Ericaceae plant species, which originates from the same family with Calluna Vulgaris (L.) Hull.

A purpose of the invention is to provide a method to obtain water-soluble regenerated fiber from Calluna Vulgaris plant species.

The invention method also aims to improve the hydrophilicity and softness of the final product via environment-friendly water-soluble yarns.

In order to achieve the above said purposes, the invention method is based on regenerated fibers obtained from Calluna Vulgaris plant.

In order to achieve the above said purposes, the invention is a method of obtaining regenerated fiber from Calluna Vulgaris plant, and it comprises the operation steps of:

- Obtaining cellulose pulp from Calluna Vulgaris plant and performing lignin disintegration,
- Performing hemicellulose disintegration in the cellulose pulp,
- Performing bleaching,
- Adding N-methyl morpholine - N-oxide, water, and polyethyleneglycol, respectively, and
- Passing the obtained solution through spinning nozzles..

In order to achieve the purposes of the invention, the operations of obtaining cellulose pulp and lignin disintegration comprise the operation steps of:

- Turning Calluna Vulgaris plant into chips,

- Keeping at ambient temperature for 8 hours via NaOH,
- baking,
- grinding,
- chemical impregnation via $Na_2SO_3$ and NaOH..

In order to achieve the purposes of the invention, the operation of hemicellulose disintegration in the cellulose pulp comprises the operation steps of:

- Adding xylanase enzyme into cellulose pulp,
- Treating cellulose pulp with xylanase enzyme at 65 °C for 150 minutes, and
- Washing cellulose pulp.

In order to achieve the purposes of the invention, during the operation of adding N-methyl morpholine - N-oxide, water, and polyethyleneglycol; 10-15 % by weight cellulose pulp is mixed with 50- 65 % by weight N-methyl morpholine - N-oxide, 10-20 % by weight polyethyleneglycol, and 10-20 % by weight water.

In order to achieve the purposes of the invention, the solution obtained following addition of N-methyl morpholine - N-oxide, water, and polyethyleneglycol, respectively, to cellulose pulp is subjected to fiber spinning by passing through spinning nozzles. Before fiber spinning operation, said solution is mixed for 20-60 minutes at 30 - 80 °C.

The structural and characteristic features of the invention and all of its advantages shall be understood better with the below given detailed description. Therefore, the assessment should be made by taking into account the detailed descriptions.

**Detailed Description of the Invention**

In this detailed description, the preferred embodiments of the water-soluble regenerated fiber production from Calluna Vulgaris plant according to the invention are only disclosed for better understanding of the subject without forming any limiting effect.

The invention relates to production of water-soluble regenerated fibers from Calluna Vulgaris (L.) Hull and Ericaceae plant species that originate from the same family.

Below, the characteristics of Calluna Vulgaris plant are given.
Biological class: Plant
Branches on the family tree: Angiosperms, Eudicots, Asterids
Regnum: Ericales
Family: Ericaceae
Genus: Calluna Salisb
Species: Calluna Vulgaris
Latin name: Calluna Vulgaris (L.) Hull

Calluna Vulgaris is a wild dwarf plant growing in acidic soil in pasture areas in cold winter months. It is a plant species that may threaten biological diversity in plateaus. It is typically a 0.5-1.25 meter high, evergreen, woody, and scrubby plant. It is pretty wooly in the beginning of growth. Then it becomes bare. It has stalkless leaves that extend through the branches; grow in four vertical lines; are green in the beginning and then become brown; and have wools up to 3.5 mm long. While its bell-shaped flowers are pale blue, they may also be pink and white. The flowers grow on the narrow leaves. Its tiny seeds are 0.5 mm long and 0.7 mm wide and in the shape of four-cell round capsules. Calluna Vulgaris spreads easily and it may occupy new areas rapidly.

Calluna Vulgaris grows new leaves and shoots beginning from spring to autumn. Its flowers are necklace-shaped and stand suspended downwards, and come into blossom towards the end of autumn. It buds out towards the end of winter. Its seeds germinate all year round. But the best germination occurs during spring and autumn. After a forest fire, it becomes capable of producing seeds in 3 years in that region. Dense green offshoots of Calluna Vulgaris plant become woody and may live about 30 years. Each plant is capable of giving one million seeds per square meter by producing 3000-4000 flowers and thousands of seeds. Its weak and tiny looking seeds survive for about 100 years. They are spread by wind or contact with animals. The seeds can even germinate only through thermal treatment without getting any light. It can also make vegetative reproduction. Calluna Vulgaris comes out in steppes, inside forest lands, and throughout tree lines. They are colonized up to 1500 meters height. They are capable of growing in wet and marsh areas; it is extremely resistant to icing.

In the below given Table 3, the development calendar of Calluna Vulgaris is given.

Table 3: Development Calendar of Calluna Vulgaris

| | Jan. | Feb. | Mar. | Apr. | May | Jun. | Jul. | Aug. | Sep. | Oct. | Nov. | Dec. |
|---|---|---|---|---|---|---|---|---|---|---|---|---|
| Blooming | | | | + | + | + | + | | | | | |
| Vegetative growth | + | + | + | + | | | | | + | + | + | + |
| Reproduction form | | | | | + | + | + | + | + | | | |
| Seeding | | | | | | + | + | + | + | + | | |
| Germination | | | + | + | + | + | + | + | + | + | + | |

Other species covered are named in Latin as Erica Cinera, Erica Lusitanica, and Erica Manipuliflora Salisb. They are found in Giresun, Trabzon, Rize, Artvin, and istanbul in Turkey.

The fact that this plant is capable of growing in acidic soils and winter months; each plant is capable of giving one million seeds per square meter by producing 3000-4000 flowers and thousands of seeds; and it can grow in marsh areas are the most significant characteristics of the wild plant, Calluna Vulgaris. It spreads easily and invades new areas quickly.

*Cellulosic substance analysis in Calluna Vulgaris plant structure via chemical methods*

*-Lignin and cellulose separation methods via acid-detergent method*

Evaluation is made for grass, hay, bush, and tree via lignin acid-detergent separation method. Cellulose is hydrolyzed in isolation with 72 % sulphuric acid ($H_2SO_4$). The reaction of the chemical substances used in the acid-detergent method leads to substantial dissolution of hemi-cellulose and allows calculating the amount of the remaining alpha-cellulose.

- CTAB (Cetyltrimethyl-ammonium bromide); 50 g of Cetyl trimethyl ammonium bromide is found in 5 litres of 0.5 Molar $H_2SO_4$.

Procedure: 100 ml of CTAB solution is added into a 250 ml beaker containing 0.500-1.000 g of dry material, and a few drops of octan-2-ol is dropped as an anti-foaming agent. The beaker is stirred for 1 hour. The obtained solution is passed through the previously weighed No. 2 sinter (W2) filter under pressure vacuum. The remaining precipitate is washed three times with boiling deionised water/acetone until it becomes colourless. The precipitate is dried at 105 °C for 2 hours, cooled in a dessicator, and weighed (W3). With this analysis method, the amount of cellulose found in C. Vulgaris plant can be determined.

$$\%\ ADF = (W3 - W2)*100 / WI$$

- Reaction chemicals: Sulphuric acid (72 % W/V) 720 ml $H_2SO_4$ is added into 540 ml deionised water.

Procedure: a sample smoothly ground with 72 % $H_2SO_4$ (15 °C) is mixed until it turns into a cake and placed in a suitable container. 3 hours later, it is washed with non-acidic water, vacuumed, dried for 2 hours at 105 C°, cooled in a dessicator, and weighed (W4). The solid portion is combusted for 2 hours in a furnace at 550 °C, cooled in a dessicator, and weighed (W5).

$$\%\ LIGNIN = (W4 - W5)*100 / WI$$

$$\%\ CELLULOSE = (W3 - W4)*100 / WI$$

With this application, % LIGNIN and % CELLULOSE amounts are determined.

In the below given Table 4, dry weight % of the cellulose found in all of the Calluna Vulgaris plant via ADF extraction is given.

Below given results are obtained with the above given two applications.

Table 4: dry weight % of the cellulose found in all of the Calluna Vulgaris plant obtained by ADF extraction

| Genus of Plant | Holocellulose (% Dry weight) | Hemi-cellulose (% Dry weight) | Alpha cellulose (% Dry weight) |
|---|---|---|---|
| Calluna Vulgaris | 36.6 | 15.2 | 21.4 |

In the below given Table 5, among the chemical compounds found in the structure of Calluna Vulgaris plant, dry weights of proteins (PRO); lipid (LIP); phenol (PHE); undefined carbohydrates (TNC); and minerals (ASH) are given in mg/g.

Table 5: Chemical analysis results of Calluna Vulgaris plant

| PRO mg/g | LIP mg/g | PHE mg/g | CEL mg/g | LIG mg/g | VAKS mg/g | TNC mg/g | ASH mg/g | TOTAL |
|---|---|---|---|---|---|---|---|---|
| 27 | 14 | 32 | 450 | 151 | 10 | 45 | 68 | 854 |

Table 5 provides the chemical analysis results of Calluna Vulgaris plant The cellulose, lignin, and fat samples forming the cellular walls are extracted in order to determine their amounts. The samples are purified by being treated in turn with the natural detergent solution formed of disodium ethylene-diaminetetra acetate dihydrate, sodium borate decahydrate, disodium hydrogen phosphate chemicals and decahydronaphthaline, acetone, and sodium sulphide.

Samples in equal amounts are treated with acid detergent solution (sulphuric acid 1 N and cetyltrimethyl ammonium bromide) so as to remove hemi cellulose. Afterwards, they are treated with potassium permanganate to remove lignin. And then, cellulose is removed by sulphuric acid (72 % W/W). Remaining oils and minerals are removed by combustion in a crematorium for 3 hours at 500 - 550 °C. The minerals are determined after keeping the material in crematorium for 5 hours at 550 °C.

Protein amount (PRO): Total nitrogen amount is determined by using Kjeldahl method.

Lipid (LIP): Determined by diethyl ether extraction for 8 hours in a Soxhlet extractor. Phenols having different molecular weights are treated at 70 °C with pure methanol and 50 % aqueous methanol solution, respectively. It is colorimetrically determined by using Folin-ciocalteu chemical substance.

For total anonymous amylase activation structures; the endo and exonuclease α- and β amylase activation is determined by using Hajedorn-jensen volumetric method following 48 hours of incubation at 37 °C.

The above-mentioned methods are general analysis methods applied on Calluna Vulgaris plant and as well as used in determination of protein, oil cellulose, lignin amounts in all plants.

In Calluna Vulgaris plant, α-amyrin, β-amyrin, oleanolic acid, taraxerol, and ursolic acid etc. compounds determined by using super critical carbondioxide extraction method are known as triterpenoids. Below, enumerated carbon frameworks of triterpenoids are given.

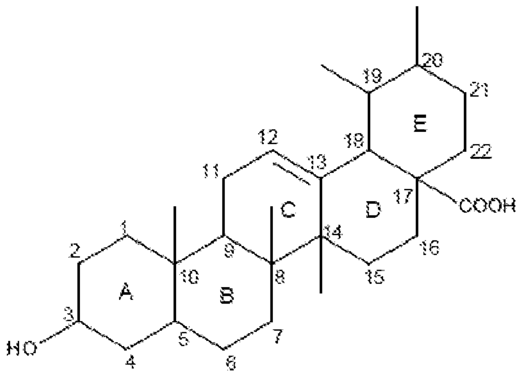

Moreover, below, triterpenoids found at the leaves, roots, and stems of Calluna Vulgaris plant are given in μg/g dry weight.

Table 6; triterpenoids found at the leaves, roots, and stems of Calluna Vulgaris plant (μg/g dry weight)

| | Leaves | Roots | Stems |
|---|---|---|---|
| α-amyrin | 35,000 | 370 | 3,900 |

(continued)

| | Leaves | Roots | Stems |
|---|---|---|---|
| β-amyrin | 13,000 | 550 | 5,700 |
| Leupeol | 19,000 | 0 | 0 |
| Oleanolic acid | 11,000 | 55 | 14,000 |
| Taraxerol | 0 | 610 | 0 |
| Taraxer-4-1 | 0 | 490 | 0 |
| Ursolic acid | 52,000 | 130 | 34,000 |
| Unnamed ursolic acid | 26,000 | 20 | 7,500 |

Biological activity of Calluna Vulgaris plant: Ursolic acid and oleanolic acid compounds show anti-cancer characteristics against leukaemia cells, gastritic tumors and lung tumors. Since it has triterpenoid compounds, it has antiulcerous and antimicrobial biological activity.

Below, the production method of water-soluble regenerated fibers from Calluna Vulgaris (L.) Hull according to the invention and Ericaceae plant species that originate from the same family.

The method of obtaining regenerated fiber from Calluna Vulgaris plant basically comprises the operation steps of:

- Obtaining cellulose pulp from Calluna Vulgaris plant and performing lignin disintegration,
- Performing hemicellulose disintegration in the cellulose pulp,
- Performing bleaching,
- Adding N-methyl morpholine - N-oxide, water, and polyethyleneglycol, respectively, and
- Passing the obtained solution through spinning nozzles..

First of all, kraft cellulose pulp is obtained from Calluna Vulgaris plant and lignin disintegration operation is performed. For obtaining this pulp, Calluna Vulgaris plant is mechanically turned into chips form. Penetration is ensured by keeping the material at ambient temperature for 8 hours with 3.0 % NaOH, and then it is baked in a pressurised container for 1 hour at 125 °C. Following baking operation, it is ground for 10 minutes in pressureless vapour. Chemical impregnation is made with 2-3 % $Na_2SO_3$ and 1-7 % NaOH, and treated for 5 minutes at 60 -120 °C. At the stage of obtaining kraft cellulose pulp, lignin disintegration is also performed in the same process.

Xylanase enzyme is applied on kraft cellulose pulp for disintegrating hemicellulose polymer structures. Enzyme dosage, 0.5 kg/ton; application time, 150 minutes; temperature, 65 °C; density, 10 % ; pH 8.5-8.7; and xylanase enzyme activity is 5000 IU/ml. The pulp treated with enzyme is then washed with distilled water.

Kraft cellulose pulp treated with xylanase enzyme is then beached with hydrogen peroxide ($H_2O_2$). Bleaching is applied on kraft cellulose pulp with 1.5 % NaOH, 0.5 % sodium silicate, 0.1 % MgSO4, 4 % $H_2O_2$, and at 90 °C and 3-5 atm pressure, for 180 minutes. The pH value and viscosity of the kraft cellulose solution (pulp) are 11 and 14.7 mPas, respectively.

The lignin amount in the pulp is found using ISO 2470 method (luminosity = 51 %) and TAPPI-T236 om-99 method (Kappa No. = 9.72).

As the lignin amount found in wood pulp solution increases, the kappa No. rises and the luminosity is reduced. More yellowish or brownish solution pulp is obtained. It is not possible to pass such a solution through spinning nozzles under pressure and obtain fibers. Since this solution will have resistance against fluidity, high pressure is required for obtaining fiber by passing the solution through spinning nozzles; but such high pressures are not possible due to the mechanical structures of the spinning nozzles. However, a pulp solution having the below given viscosity, kappa value, and luminosity characteristics would optimally allow performing fiber spinning.

1. Viscosity: 14.7 mPas
2. Kappa No. = 9.72
3. Luminosity = 51 %

55 % amine oxide NMMO, 20 % PEG, 10 % water, 15 % cellulose pulp mixture are prepared in a container and mixed at 30-70 °C at 40 RPM for 60 minutes. The preferred rates in said mixture are given below in table form.

Table 7: PEG/NMMO/Water/Cellulose mixing rates

| Ingredient | Preferred amount by weight (%) | Usable amount by weight (%) |
|---|---|---|
| Cellulose pulp obtained from Calluna Vulgaris plant | 15 | 10-15 |
| N-methylmorpholine - N-oxide (NMMO) | 55 | 50-65 |
| Water | 10 | 20-10 |
| Polyethyleneglycol (PEG) | 20 | 20-10 |

Cellulose - Amine Oxide (NMMO) - Polyethyleneglycol (PEG) solution: 10-15 % cellulose, 50-65 % amine oxide (NMMO), 10-20 % PEG, and 10-20 % water. Through the spinning nozzles; cellulose solution completes molecular orientation via wet spinning method.

With wet fiber spinning method; a solution consisting of polyethylene glycol, NMMO, cellulose pulp, and water is mixed for 20-60 minutes at 30 - 80 °C, and then fiber spinning is performed by using spinning nozzles made of stainless steel, nickel, or platinum alloys with nozzle channel diameters ranging between 0.2 - 1 mm.

In the cellulose pulp obtained from Calluna Vulgaris plant, the lignin material is required to be removed completely. Viscosity of polyethylene glycol / water / cellulose / NMMO solution should be fluid such that it would not prevent formation of yarn. The fiber diameter increases as the viscosity increases.

While cellulose is mixed with 10-20 % PEG and 50-65 % aqueous amine oxide (NMMO) solution, cellulose activation temperature increases up to 60 °C. Cellulose swells in polar media such as water. The temperature of swollen cellulose increases up to 70 °C in amine oxide (NMMO)-water mixture. If this temperature is maintained for about 60 minutes, cellulose activation is completed. In this way, good mixture and homogeneous cellulose melt are obtained. Before the formed solution turns into solid chips and granule form, subvention can be made to the spinning part. Homogeneous polymer melt or solution is a key product having smooth spinning performance and final product characteristics.

Different from the traditional raw material sources of other regenerated fibers, within the scope of the invention, regenerated fiber is obtained by using Calluna Vulgaris (L.) Hull and Ericaceae plant species from the same family. As the organic solvent, N-methyl morpholine - N-oxide is used. Polyethylene glycol polymer structure that can form amorphous groups is preferred. Viscosity is reduced by using polyethylene glycol polymer. Moreover, the hydrogen bridge bonds among the cellulose macro molecules are reduced by using a polyethylene glycol polymer structure. Polyethylene glycol polymer is water dissoluble. The yarn obtained from regenerated fibers according to the invention method can be dissolved in water by using cellulose enzymes or acidic solutions. Attraction force occurs between each solution having partial electronegative charge $\delta$- and hydrogen atoms found in cellulose macro molecules and having partial positive $\delta$+ charge. Bond polarity increases with the partial attractive forces between the hydrogen groups having partial positive $\delta$+ charge in the solution polymer also containing the solvent substance and the oxygen or halogen groups in the solvent substance having negative charge.

Cellulose material found in Calluna Vulgaris plant is utilized as cellulose raw material source and water-soluble polymer yarn can be obtained from this raw material by using regenerated cellulose fiber obtaining methods. Hydrophilicity and softness of the final product are improved with environment-friendly water-soluble yarns. Water-soluble fibers cannot be obtained with traditional regenerated cellulose fiber obtaining methods. Thanks to the invention, water-soluble polymer fiber can be produced via regenerating method by using renewable cellulose source. Moreover, water-soluble regenerated cellulose fibers can be re-used as cellulose polymers via recycling process. Conventional PVA polymer known as water-soluble polymer is separated into acetaldehyde or formaldehyde molecules as a result of dissolving in acetic acid or formic acid at high temperatures, and cannot be used in recycling processes.

Cellulose pulp obtained after removing lignin, pectin etc. substances from Calluna Vulgaris plant containing 21.4 % cellulose is treated with N-methylmorpholine - N-oxide solvent, and thus modified fiber is obtained. Since the degree of polymerization of cellulose polymers forming these fibers are low, they dissolve in water when they are treated with cellulose enzyme. Therefore, the fibers according to the invention method can be used in place of PVA (polyvinyl alcohol).

Different from natural fibers, cellulose macro molecules forming regenerated cellulose fiber have low average polymerization (OP) degrees. OP degrees of cellulose materials or linters used as raw material in obtaining regenerated cellulose fibers are actually not that low. However, as the polymerization degrees of macro molecules increase, the viscosities of their solutions also increase in parallel. Since very high pressure is required for passing high viscosity solutions smoothly through small nozzle holes, cellulose macro molecules are partially disintegrated while preparing fiber solution. The polymerization degrees of cellulose macro molecules used for obtaining fibers is required to be above 200.

As a significant result of seeing low average polymerization degrees in cellulose macro molecules that form

normal regenerated cellulose fibers, these fibers have much lower wet strength compared to dry strength (wet breaking strength is about 55-65 % of dry breaking strength).

Polyethyleneglycol polymer structure is formed of hydrogen bonds between cellulose macromolecules and N-methyl morpholine within fiber spinning solution. In this way, since the strength of hydrogen bonds and Vander Waals attraction forces among cellulose macro molecules would be reduced, the wet strength of obtained fibers is lower. Therefore, they are dissolved easily in water by using cellulose enzymes in dye - treatment processes.

Since polyethyleneglycol reduces viscosity, the hydrogen bonds and Vander Waals attraction forces are reduced among cellulose macro molecules. As a result, weaker macromolecular cellulose polymer is obtained. Since the amorphous region of said fibers is 30-40 % more than traditional regenerated fibers and they have lower wet strength, they can easily be dissolved in cellulose enzyme solution at 50 °C - 60 °C temperature. Already short cellulose macro molecules of regenerated cellulose fibers cannot be placed uniformly within the fiber. Therefore, a big portion of these fibers (about 80-85 %) is formed of amorphous regions. Non-uniform arrangement of macro molecules reduces the attractive forces among these molecules as well as reducing their wet strength, and at the same time, causes their water and aqueous flottes to impact on amorphous regions in a simpler and more effective manner.

In spinning bath, polyethyleneglycol, NMMO, and cellulose solution is oriented with wet spinning method in an extruder at 30 - 80 °C te mperature range.

Below, a reaction scheme is given, which shows that polyethyleneglycol molecule forms hydrogen bonds between NMMO and cellulose macro molecules and thus prevents formation of hydrogen bridge bonds between the solvent (N-methyl morpholine N-oxide) and cellulose macro molecules, and allows formation of amorphous structures among cellulose macro molecules.

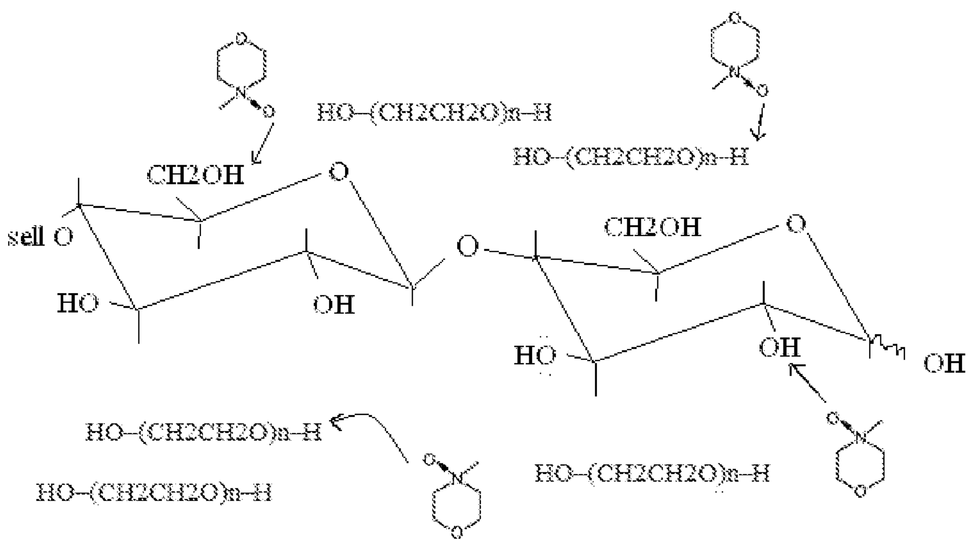

HO-(CH2CH2O)n-H = Polyethylene glycol

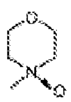 = NMMO; N methyl morpholine N-oxide

Amorphous regions are formed among cellulose macro molecules by using 10-20 % polyethylene glycol polymer. During the dyeing process of this yarn having lower wet strength, its removal by using cellulose enzyme is much easier. In this way, yarns obtained from cellulose raw material with this method become water soluble. Polyethylene glycol polymer is an easily water soluble polymer. It is an organic product compared to obtaining process steps of polyvinyl alcohol yarn. Polyvinyl alcohol yarn commonly used in textile sector is used in specific processes due to is water soluble characteristics. Yet, hydrocotton products can also be referred to as organic production, since regenerated cellulose yarn obtained with polyethylene glycol process is used instead of polyvinyl alcohol yarn, the regenerated cellulose yarn is an organic product, and the process method is suitable.

Polyethylene glycol polymer forms hydrogen bonds between cellulose macro molecules during fiber spinning. By means of these hydrogen bonds, while their dry strength reaches 7-9.5 g/denier levels, their wet breaking strength is reduced down to 3-5.5 g/denier levels. Their specific weight is around 1.35-1.45 g/$cm^3$.

During application, the yarns obtained with the method according to the invention are not required to be treated

with formic acid or acetic acid at 90-100 °C temperature during additional bath before bleachery and dyeing, as in removal processes of PVA yarns from raw materials. Application of pilling operation found in dyeing process is adequate instead of this process. In the pilling operation, cellulose enzyme is applied at 2-3 g/L, pH 5-5.5, and 50-60 °C for 30 minutes.

It is possible to use polyvinylchloride polymer structure instead of polyethylene glycol. Hydrogen bridge bonds can be formed between cellulose macro molecules by forming amorphous groups with this polymer. Moreover, wet or dry spinning processes can be developed by using polyvinylchloride/NMMO/water solvents. Accordingly, following obtaining of yarn via fiber spinning from polymers that can form hydrogen bridge bonds with cellulose macro molecules, yarns that can easily be removed in water can be obtained by using enzyme or solvents.

An attraction force occurs between each polymer having partial electronegative charge $\delta$- and hydrogen atoms found in cellulose macro molecules and having partial positive $\delta$+ charge.

NMMO/$H_2O$/PEG are used as solvents. Regenerated cellulose fiber is obtained by forming cellulose solution, NMMO determined as amine-oxide, and the yarns obtained from these fibers are made water-soluble using cellulose enzyme.

## Claims

1. A method of obtaining regenerated fiber from Calluna Vulgaris plant, and it is **characterized in that**; it comprises the operation steps of:

   • Obtaining cellulose pulp from Calluna Vulgaris plant and performing lignin disintegration,
   • Performing hemicellulose disintegration in the cellulose pulp,
   • Performing bleaching,
   • Adding N-methyl morpholine - N-oxide, water, and polyethyleneglycol, respectively, and
   • Passing the obtained solution through spinning nozzles..

2. The method according to Claim 1, **characterized in that**; the operations of obtaining cellulose pulp and lignin disintegration comprise the operation steps of:

   • Turning Calluna Vulgaris plant into chips,
   • Keeping at ambient temperature for 8 hours via NaOH,
   • baking,
   • grinding,
   • chemical impregnation via $Na_2SO_3$ and NaOH..

3. The method according to Claim 1, **characterized in that**; the operation of hemicellulose disintegration in the cellulose pulp comprises the operation steps of:

   • Adding xylanase enzyme into cellulose pulp,
   • Treating cellulose pulp with xylanase enzyme at 65 °C for 150 minutes, and
   • Washing cellulose pulp..

4. The method according to Claim 1, **characterized in that**; during the operation of adding N-methyl morpholine - N-oxide, water, and polyethyleneglycol; it comprises the operation step of mixing 10-15 % by weight cellulose pulp with 50- 65 % by weight N-methyl morpholine - N-oxide, 10-20 % by weight polyethyleneglycol, and 10-20 % by weight water.

5. The method according to Claim 1, **characterized in that**; it comprises the operation step of performing fiber spinning by passing the solution obtained following addition of N-methyl morpholine - N-oxide, water, and polyethyleneglycol, respectively, to cellulose pulp, through spinning nozzles.

6. The method according to Claim 5, **characterized in that**; before fiber spinning operation, it comprises the operation step of mixing said solution for 20-60 minutes at 30 - 80 °C.

## Patentansprüche

1. Verfahren zum Erhalten von regenerierter Faser der Pflanze Calluna Vulgaris, das **dadurch gekennzeichnet ist,**

**dass** es die folgenden Vorgangsschritte umfasst:

• Erhalten von Cellulosepulpe der Pflanze Calluna Vulgaris und Durchführen von Desintegration von Lignin,
• Durchführen von Desintegration von Hemicellulose in der Cellulosepulpe,
• Durchführen von Bleichung,
• jeweils Hinzufügen von N-Methylmorpholin-N-Oxid, Wasser und Polyethylenglycol, und
• Geben der erhaltenen Lösung durch Spinndüsen.

**2.** Verfahren nach Anspruch 1, **dadurch gekennzeichnet, dass** die Vorgänge des Erhaltens von Cellulosepulpe und der Desintegration von Lignin die folgenden Vorgangsschritte umfassen:

• Umwandeln der Pflanze Calluna Vulgaris in Chips,
• 8 Stunden Halten bei Umgebungstemperatur über NaOH,
• Backen,
• Mahlen,
• chemische Imprägnierung über $Na_2SO_3$ und NaOH.

**3.** Verfahren nach Anspruch 1, **dadurch gekennzeichnet, dass** der Vorgang der Desintegration von Hemicellulose in der Cellulosepulpe die folgenden Vorgangsschritte umfasst:

• Hinzufügen von Xylanaseenzym in die Cellulosepulpe,
• 150-minütiges Behandeln der Cellulosepulpe mit Xylanaseenzym bei 65 °C, und
• Waschen der Cellulosepulpe.

**4.** Verfahren nach Anspruch 1, **dadurch gekennzeichnet, dass** es während des Vorgangs des Hinzufügens von N-Methylmorpholin-N-Oxid, Wasser und Polyethylenglycol den Vorgangsschritt des Vermischens von 10-15 Gewichts-% Cellulosepulpe mit 50-65 Gewichts-% N-Methylmorpholin-N-Oxid, 10-20 Gewichts-% Polyethylenglycol und 10-20 Gewichts-% Wasser umfasst.

**5.** Verfahren nach Anspruch 1, **dadurch gekennzeichnet, dass** es den Vorgangsschritt des Durchführens von Faserspinnen umfasst, indem die Lösung, die nach dem Hinzufügen jeweils von N-Methylmorpholin-N-Oxid, Wasser und Polyethylenglycol zu Cellulosepulpe erhalten wird, durch Spinndüsen gegeben wird.

**6.** Verfahren nach Anspruch 5, **dadurch gekennzeichnet, dass** es vor dem Vorgang des Faserspinnens den Vorgangsschritt des 20-60-minütigen Vermischens der Lösung bei 30-80 °C umfasst.

## Revendications

**1.** Procédé d’obtention de fibre régénérée à partir d’une plante Calluna Vulgaris, et il est **caractérisé en ce que** : il comprend les étapes d’opération :

• d’obtention de pulpe de cellulose d’une plante Calluna Vulgaris et de réalisation d’une désintégration de la lignine,
• de réalisation d’une désintégration de l’hémicellulose dans la pulpe de cellulose,
• de réalisation d’un blanchiment,
• d’ajout de N-méthylmorpholine-N-oxyde, d’eau, et de polyéthylèneglycol, respectivement, et
• de passage de la solution obtenue à travers des buses de filage.

**2.** Procédé selon la revendication 1, **caractérisé en ce que** ; les opérations d’obtention de pulpe de cellulose et de désintégration de la lignine comprennent les étapes d’opération :

• de transformation de la plante Calluna Vulgaris en copeaux,
• de maintien à température ambiante pendant 8 heures avec du NaOH,
• de cuisson,
• de broyage,
• d’imprégnation chimique avec du $Na_2SO_3$ et du NaOH.

3. Procédé selon la revendication 1, **caractérisé en ce que** ; l'opération de désintégration de l'hémicellulose dans la pulpe de cellulose comprend les étapes d'opération :

- d'ajout d'enzyme xylanase dans la pulpe de cellulose,
- de traitement de la pulpe de cellulose avec l'enzyme xylanase à 65 °C pendant 150 minutes, et
- de lavage de la pulpe de cellulose.

4. Procédé selon la revendication 1, **caractérisé en ce que** ; pendant l'opération d'ajout de N-méthylmorpholine-N-oxyde, d'eau, et de polyéthylèneglycol ; il comprend l'étape d'opération de mélange de 10 à 15 % en poids de pulpe de cellulose avec 50 à 65 % en poids de N-méthylmorpholine-N-oxyde, 10 à 20 % en poids de polyéthylèneglycol, et 10 à 20 % en poids d'eau.

5. Procédé selon la revendication 1, **caractérisé en ce que** ; il comprend l'étape d'opération de réalisation de filage de fibre en passant la solution obtenue à la suite de l'ajout de N-méthylmorpholine-N-oxyde, d'eau, et de polyéthylèneglycol, respectivement, à la pulpe de cellulose, à travers des buses de filage.

6. Procédé selon la revendication 5, **caractérisé en ce que** ; avant l'opération de filage de fibre, il comprend l'étape d'opération de mélange de ladite solution pendant 20 à 60 minutes à une température allant de 30 à 80 °C.